# EUROPEAN PATENT APPLICATION

(11) **EP 2 952 208 A1**
(43) Date of publication of application: **09.12.2015**
(21) Application number: 14382215.3
(22) Date of filing: 04.06.2014
(51) Int. Cl.: A61K 47/10, A61K 47/34, A61K 9/00

(54) **Hydroalcoholic system for nail treatment**

(71) Applicant: Universidade de Santiago de Compostela, 15782 Santiago de Compostela (ES)
(72) Inventor: Otero Espinar, Francisco J, 15782 Santiago de Compostela (ES); Anguiano Igea, Soledad F, 15782 Santiago de Compostela (ES); Cutrín Gómez, Elena, 15782 Santiago de Compostela (ES); Gómez Amoza, José Luis, 15782 Santiago de Compostela (ES)
(74) Representative: Lorca Melton, Miguel

(57) **Abstract**

The present invention refers to A pharmaceutical composition comprising (i) poloxamer 407, (ii) at least one penetration enhancer, (iii) at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof.

## Description

### Field of the Invention

The present invention relates to drug delivery systems for the treatment of nail diseases. In particular, the invention relates to the preparation of a hydroalcoholic composition and its use in the manufacture of medicaments.

### Background of the Invention

Pathological nail disorders may include relatively harmless conditions such as pigmentation changes, which commonly occur in smokers, discoloration associated with the use of certain systemically administered drugs, or increased fragility (e.g. by the continued use of detergents). Nevertheless, nail disorders may be more serious, accompanied by painful, debilitating processes, dystrophy, hypertrophy and inflammatory or infectious processes. These conditions can affect patients negatively from a physical standpoint and are accompanied by an important social and psychological component that can degrade the quality of life.

Onychomycosis (fungal infection that affects 3-10% of the population in Europe) and psoriasis (autoimmune illness, which is suffered by the 1-3 % of population) are the main diseases that alter nail plate. In psoriasis and onychomycosis, the promotion of the drug penetration (steroids and antifungal agents) by using appropriate formulations may improve drug efficacy with a consistent reduction of side effects. In this sense, the main strategies currently used to improve the effectiveness of local treatment are (Baran R, Tosti A. Topical treatment of nail psoriasis with a new corticoid-containing nail lacquer formation. Journal of Dermatological Treatment. 10, 201-204, 1999 ; Monti D, Saccomani L, Chetoni P, Burgalassi S, Saettone MF, Mailland F. In vitro transungual permeation of ciclopirox from a hydroxypropyl chitosan-based, water-soluble nail lacquer. Drug Development and Industrial Pharmacy. 1:11-17, 2005; Mohori M, Torkar A, Friedrich J, Kristl J, Murdan S. A investigation into keratinolytic enzymes to enhance ungual drug delivery. International Journal of Pharmaceutics. 332, 196-201, 2007; Hui X, Shainhouse Z, Tanojo H, Anigbogu A, Markus GE, Maibach HI, Wester RC. Enhanced human nail drug delivery: nail inner drug content assayed by new unique method. Journal of Pharmaceutical Sciences. 91, 189-195, 2002 ; Hui X, Wester R.C, Barbadillo S, Lee C, Patel B, Wortzmman M, Gans EH, Maibach HI. Ciclopirox delivery into the human nail plate. Journal Of Pharmaceutical Sciences. 93, 2545-2548, 2004):
- Use of more potent drugs.
- Selection of drugs with suitable physicochemical properties to facilitate their penetration and diffusion into the nail and nail matrix
- Use of penetration and diffusion enhancers. (e.g., U.S. Patent No. 6,042,845, 6,159,977, 6,224,887, 6,391,879).
- Design of formulations that bear high drug concentration and with prolonged residence times on the nail plate to promote a controlled release of the drug.

Sun et al. concluded that the main problem of topical treatment is related to unsuitable formulations and poor drug release (Sun Y, Liu JC, Wang JC T, De Doncker P. Nail penetration. Ontopical Focus delivery of drugs for onychomycosis fungal Treatment. In: Bronaugh, RL, Maibach, HI (Eds), Percutaneus absorption. Drugs-Cosmetics-Mechanims-Methodology, 3rd Ed Marcel Dekker Inc, New York, pp. 759-787, 1999).

In this way some topical antifungal nail lacquers, which increase both the residence time of the vehicle and drug penetration, were marketed in recent years: Loceryl^{®} or Odenil^{®} (amorolfine 5% by Galderma SA), CICLOCHEM^{®} (ciclopirox by Novag Laboratories), or Penlac^{®} Nail Lacquer (ciclopirox by Sanofi Aventis).

Further examples of ungual drug delivery systems described in the patent literature are the following patent documents: US 4,957,730 (describes a solution of 1-hydroxy-2-pyridone that generates a water resistant film); US 5,120,530 (amorolfine in a quaternary ammonium acrylic copolymer); US 5,264,206 (tioconazole, econazole, oxiconazole, miconazole, tolnaftate, naftilina hydrochloride, included in a film insoluble in water); US 5,346,692 (with urea and dibutyl phthalate as plasticizer) ; US 5,487,776 (griseofulvin as colloidal suspension), US 5,464,610 (plaster of salicylic acid); WO 1999/39680 (nail lacquer with dioxanes, dioxolanes y acetals as penetration enhancers), US 6,495,124 (antifungal nail lacquer elaborated with filmogenic polymers dispersed in organic solvents including cyclic lactones as plasticizer and penetration enhancers).

The effectiveness of the above nail lacquers (based on organic solvents) as vehicles for topical administration of an antifungal agent, amorolfine, has already been described by Jean-Paul L. Marty, J. European Academy of Dermatology and Venereology, 4 (Suppl. 1), pp. S17-S21 (1995). However, nail lacquers are usually composed of polymers dispersed or dissolved in volatile organic solvents leading to the formation of highly viscous, water-impermeable films on the nail plate surface as solvent evaporates. Nevertheless, the use of organic solvents exhibits important drawbacks such as their toxicity, irritation, low diffusion of drugs and enhancers and occlusive, harmful effects in fungal infections. With the aim of minimizing these effects, it has been proposed to replace organic nail lacquers with aqueous formulations. In these systems water solutions or mixtures of water with cosolvents are used as drug vehicle in the preparation of nail lacquers. Examples of this group of formulations are US 2009/0175945, antipsoriatic nail lacquer containing mixtures of water and alcohol, nail polish EP 039132 containing acrylic polymer dissolved in water; EP 0 627 212 aqueous coating containing a polyurethane polymer film forming and an organic compound soluble perfluoroalkyl type; EP 0 648 485 lacquer containing aqueous anionic polyester-polyurethane polymer in dispersed state, EP 0 943 310 or US 6,238,679 film-forming composition comprising an aqueous polyurethane dispersion and an agent plasticizer and US 7,033,578 (nail varnish made from chitosan derivatives in volatile solvents).

There is thus an ongoing interest in providing new improved compositions for the treatment of ungual conditions.

It has been shown that water hydrates and swells nail structures promoting the penetration and diffusion of drugs (Gunt HB, Kasting GB. Effect of hydration on the Permeation of ketoconazole through in vitro human nail plate. 32, 254-260, 2007; Gunt HB, Miller MA, Kasting GB. Water diffusivity in human nail plate. Journal of Pharmaceutical Sciences. Vol. 96, No. 12, 3352-3362, 2007). For these reasons aqueous systems have been proposed as ungual delivery systems: gels (semisolid), hydrogels, creams or aqueous lacquers. Examples of patents are WO 2009/089361 (hydrogels containing several layers for controlling the release), US 2009/0202602 (patches made from crosslinked hydrogels of alkyl-pyrrolidone), US 5,696,105 (mometasone furoate cream).

Thus, aqueous systems have attracted most efforts in recent years. Most drugs however have a poor solubility in water and further improvements have been achieved by the incorporation of cyclodextrins. It has been shown that the incorporation of free cyclodextrins or cyclodextrins:polymers complexes increases drug aqueous solubility in the aqueous systems, increasing the effective drug load that can be included in soluble form and significantly reducing the strength of the aqueous layer (Bibby DC, Davies NM, Tucker IG. Mechanisms by which cyclodextrins modify drug release from polymeric drug delivery systems. International Journal of Pharmaceutics. 197, 1-11, 2000; Brewster ME, Loftsson T. Cyclodextrins as pharmaceutical solubilizers. Advanced Drug Delivery Reviews 59, 645-666, 2007). The inventors themselves have obtained excellent results with a thermosensitive aqueous hydrogel combining poloxamer 407, a drug, a penetration enhancer and a cyclodextrin or a water soluble polymer.

In fact, a large body of evidence has been built demonstrating the benefits of aqueous formulations and the drawbacks of incorporating organic solvents (e.g. alcohols or esters) due to their deleterious effect in nail hydration and swelling. However, high nail swelling is usually associated with improved drug penetration and it is known that organic solvents dehydrate the nail matrix. They are typically used only when mandatory due to solubility issues (e.g. US 5,391,367). Early studies support this notion. Mertin D, Lippold BC. J Pharm Pharmacol., 1997; 49, 30-34 showed how diluted alcohol solutions provide better drug penetration than the corresponding neat solutions due to increased nail hydration. Further, Quintar-Guerrero, D. et al, Drug Dev. Ind. Pharm., 1998, 24(7), 685-90 found that the presence of ethanol (used as co-solvent) did not promote the passage of antimycotics. According to the authors, although ethanol had been reported as a skin permeation enhancer, it did not appear to have the same effect on the nail; the corneocytes are joined in a tightly cemented continuous sheet, with overlap of their borders, which constituted a barrier that was insensitive to the effect of ethanol. In Gouri, V.G. et al, J. Comet. Sci., 1999, 50, 363-85 the effect of chain length in the alcohol was discussed, pointing to a decreased drug penetration in the nail with increased chain length.

More recent studies point in the same direction. Smith et al. J Pharm Sci., 2011 May 23, "Effects of Organic Solvents on the Barrier Properties of Human Nail*"* studies the effect on the ungual barrier of incorporating in aqueous compositions organic solvents such as ethanol, propylen glycol (PPG) or poliethylene glycol 400 (PEG). The study shows how these three organic solvents reduce diffusion and penetration through the nail. Permeant partitioning into and transport across the nail were shown to decrease as the concentration of the organic solvent in the binary solvent system increased.

Thus, improved compositions for drug delivery to and into nails are still needed. Providing delivery systems with increased penetration and diffusion improves the effectiveness of the treatment and reduces side effects. Such composition would preferably show good bioadhesiveness and long acting effectiveness. Also, compositions with better cosmetic results and high drying speed are desirable in order to provide further comfort and increase patient compliance.

### Brief Description of the Invention

The present invention provides a hydroalcoholic pharmaceutical system for the administration of drugs to nails which improves current products. The composition of the invention is surprisingly effective, providing an expected nail penetration and diffusion. Contrary to current established knowledge, the inventors have found that the use of a short chain alcohols in the aqueous composition of the invention does not reduce drug delivery properties, but surprisingly increases penetration and diffusion of the drug to and into the nail. The composition of the invention provides further advantages discussed below.

Thus, a first aspect of the invention is a pharmaceutical composition comprising (i) poloxamer 407, (ii) at least one penetration enhancer, (iii) at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof.

A further aspect of the invention is a kit comprising a pharmaceutical composition which comprises (i) poloxamer 407, (ii) at least one penetration enhancer, (iii) at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof; and instructions for ungual application of said pharmaceutical composition.

Contrary to common understanding, the hydroalcoholic composition of the invention provides better nail penetration and diffusion of the corresponding biologically active substance than the corresponding aqueous system.

Further aspects of the invention are directed to
- A pharmaceutical composition as defined above for used as a medicament.
- A pharmaceutical composition as defined above for use in the treatment and prevention of ungual conditions.
- A Method for the preparation of a pharmaceutical composition which comprises mixing (i) poloxamer 407, (ii) at least one penetration enhancer, (iii) at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof.

### Brief description of the figures

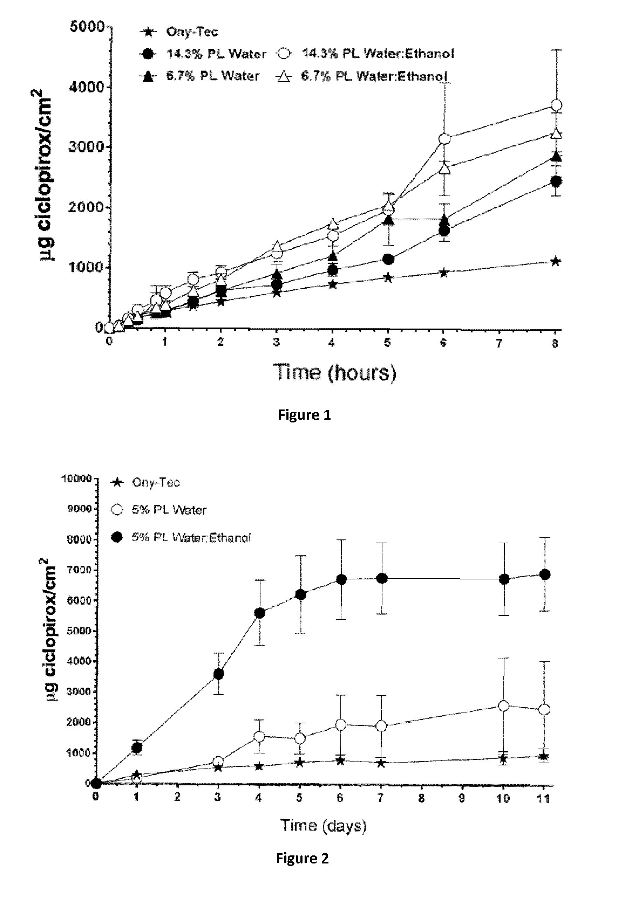

**Figure 1** shows the comparison in release profile between formulations having different poloxamer 407 proportions (PL), CRYSMEB in 10% w/w and N-acetylcisteine in 10% w/w, using hydroalcoholic solutions, and the corresponding solutions with water as only vehicle, and also compared to the commercial formulation Ony-Tec. 0.5ml of solution were placed in the donor compartment.
**Figure 2** shows the comparison in diffusion profile of ciclopirox olamine through bovine hoof between (A) a formulation according to the invention comprising water:ethanol (1:1); (B) Ony-Tec (comparative); and (C) a formulation comprising water as only vehicle (comparative). Compositions (A) and (C) comprise poloxamer 407 5% w/w, CRYSMEB in 10% w/w and N-acetylcisteine in 10% w/w. 0.5ml of solution were placed in the donor compartment.

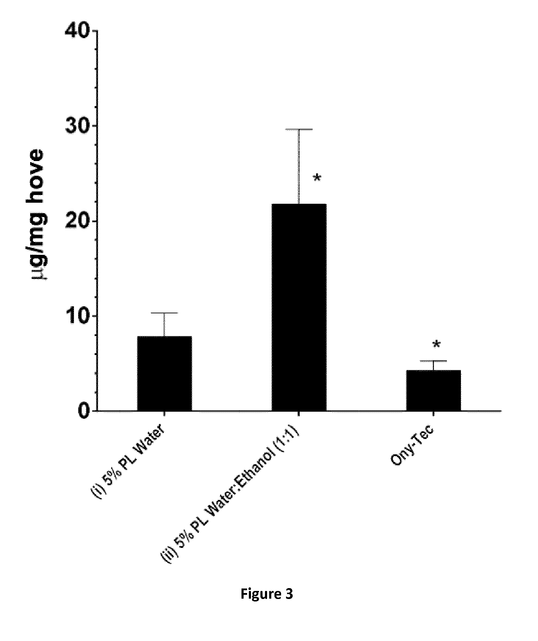

**Figure 3** shows the levels of ciclopirox olamine inside the bovine hoof after 11 days of diffusion testing with 0.5 ml of (A) a formulation according to the invention comprising water:ethanol (1:1); (B) Ony-Tec (comparative); and (C) a formulation comprising water as only vehicle (comparative). Compositions (A) and (C) comprise poloxamer 407 5% w/w, CRYSMEB in 10% w/w and N-acetylcisteine in 10% w/w. Asterisk indicates that statistically significant differences were found for á<0,05 (one way ANOVA and multiple comparison Tukey test).

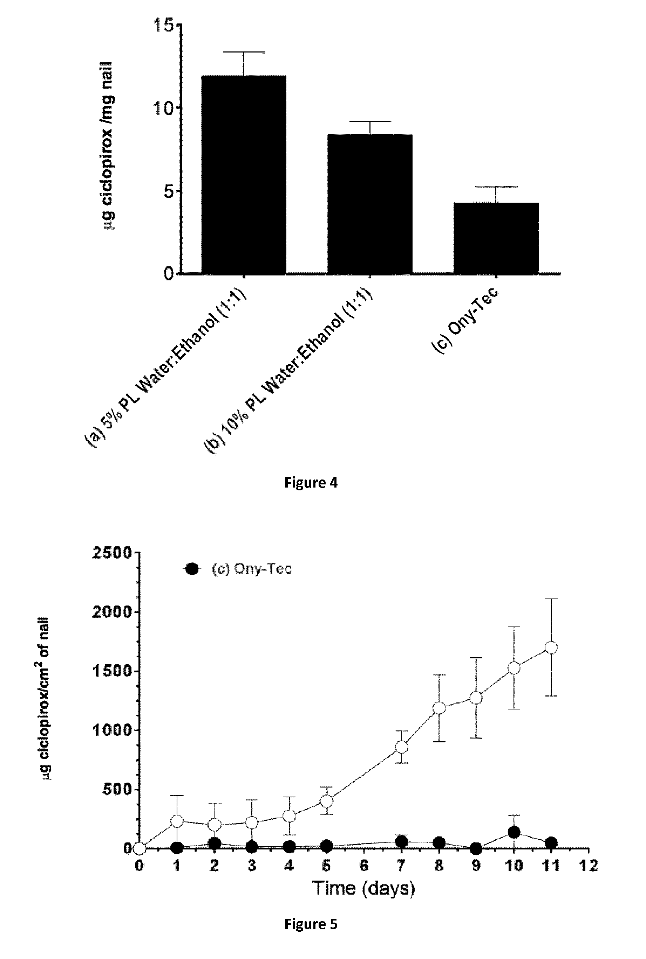

**Figure 4** shows the levels of ciclopirox olamine inside human nail after 11 days of diffusion testing with the following formulations (a) 5% w/w poloxamer 407, 10% w/w of N-acetylcistein, 10% w/w CRYSMEB, water:ethanol (1:1) and 24.27mg/ml of ciclopirox olamine; and (b) 10% w/w of poloxamer 407, 10% w/w of N-acetylcistein, 10% w/w CRYSMEB, water:ethanol (1:1) and 22,90 mg/ml of ciclopirox olamine, as well as a solution (c) of Ony-Tec (comparative). In order to perform the test, 0.5 ml of solution were placed in the donor compartment on the first day, and 0.5 ml on the fifth day.
**Figure 5** shows the diffusion of ciclopirox olamine through human nail of the following compositions (a) 5% w/w of poloxamer 407, 10% w/w of N-acetylcistein, 10% w/w CRYSMEB, water:ethanol (1:1) and 24.27mg/ml of ciclopirox olamine, compared to a composition of Ony-Tec (comparative). 2 ml of solution were placed in the donor compartment.

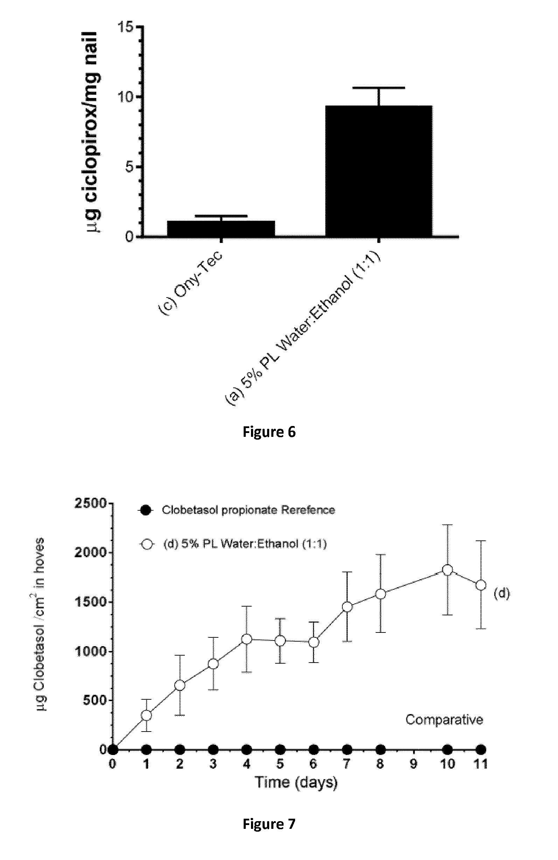

**Figure 6** shows the levels of ciclopirox olamine inside human nail after 11 days of diffusion testing with formulations comprising (a) 5% w/w of poloxamer 407, 10% w/w of N-acetylcistein, 10% w/w CRYSMEB, water:ethanol (1:1) and 24.27mg/ml of ciclopirox olamine, compared to a compositon of Ony-Tec (comparative). 2 ml of solution were placed in the donor compartment.
**Figure 7** shows the diffusion of clobetasol propionate through bovine hoof of the following composition (d) 5% w/w of poloxamer 407, 10% w/w of N-acetylcistein, 10% w/w CRYSMEB, water:ethanol (1:1) and 1,6 mg/ml of clobetasol propionate, compared to a composition comprising 80 mg/ml of clobetasol propionate using FAGRON as base formulation (comparative). 0.5 ml of solution were placed in the donor compartment.

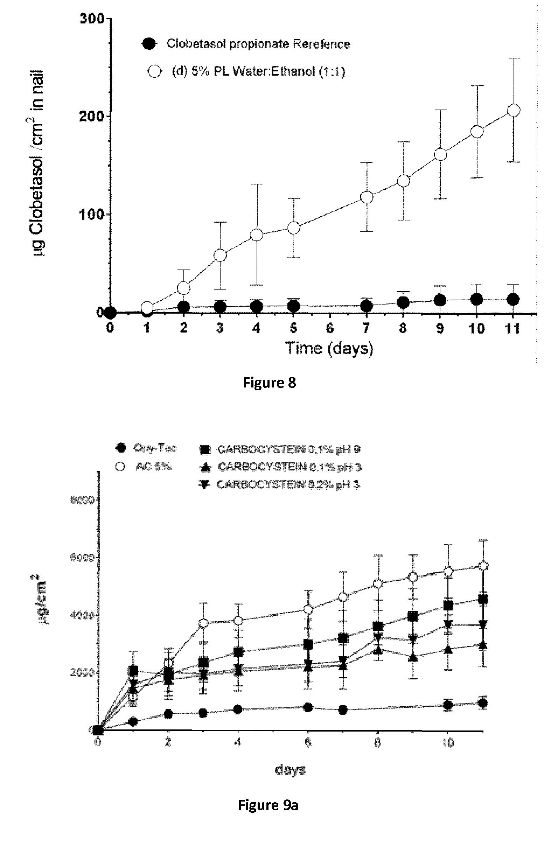

**Figure 8** shows the diffusion of clobetasol propionate through human nail of the following composition comprising (d) 5% w/w of poloxamer 407, 10% w/w of N-acetylcistein, 10% w/w CRYSMEB, water:ethanol (1:1) and 1,6 mg/ml of clobetasol propionate, compared to a composition comprising 80 mg/ml of clobetasol propionate using FAGRON as base formulation (comparative). 0.5 ml of solution were placed in the donor compartment.

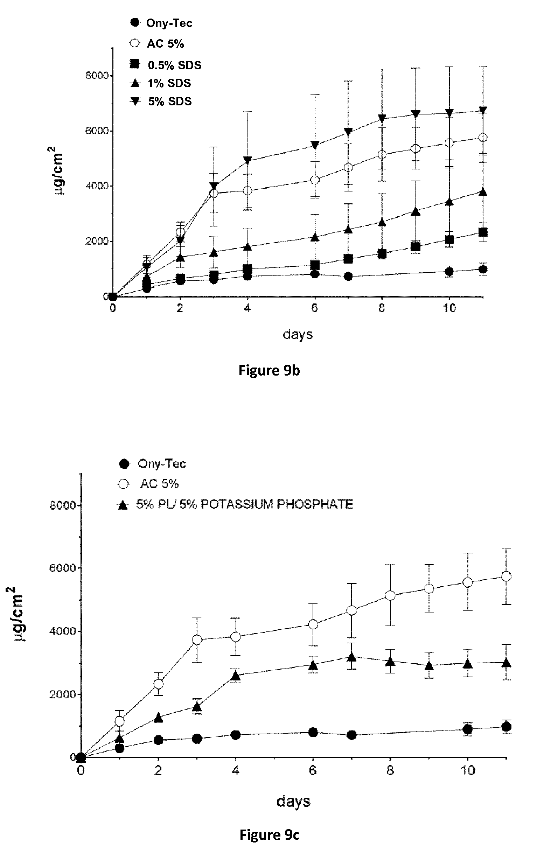

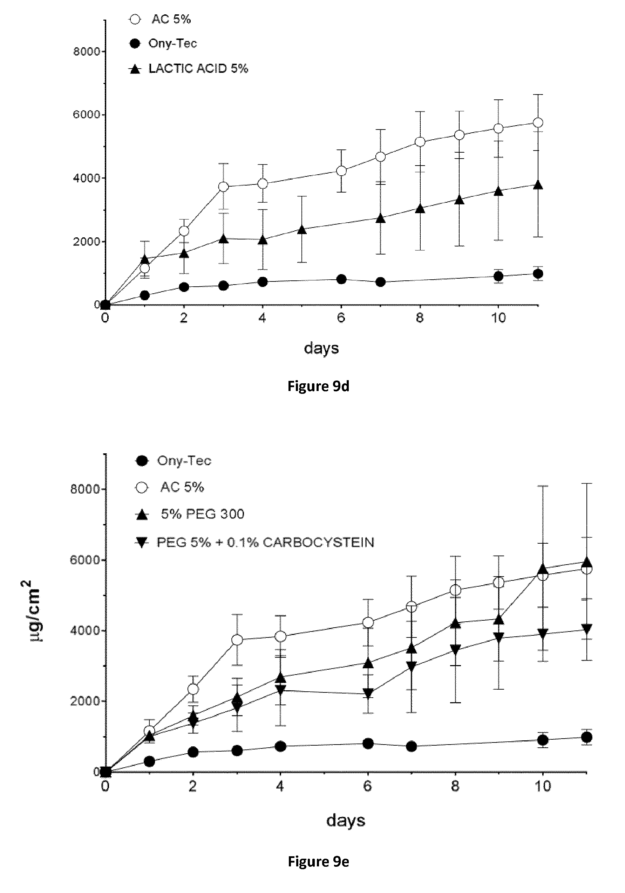

**Figures 9a****-e** show ciclopiroxolamine diffusion across bovine hoof membrane of pharmaceutical compositions of the invention (2 ml) prepared with different nail penetration enhancers (mean and standard error, n=3). Ony-tech was used as a comparison and pharmaceutical compositions according to the invention having N-acetyl-cisteine (Ac) were used as reference. Figure 9a shows results of carbocysteine. Figure 9b shows the results of Sodium Lauryl sulfate (SLS). Figure 9c shows the results of potassium phosphate. Figure 9d shows the results of lactic acid. Figure 9e shows the results of PEG300 and PEG300 in combination with carbocystein. In all cases the pharmaceutical compositions of the invention provided good results.

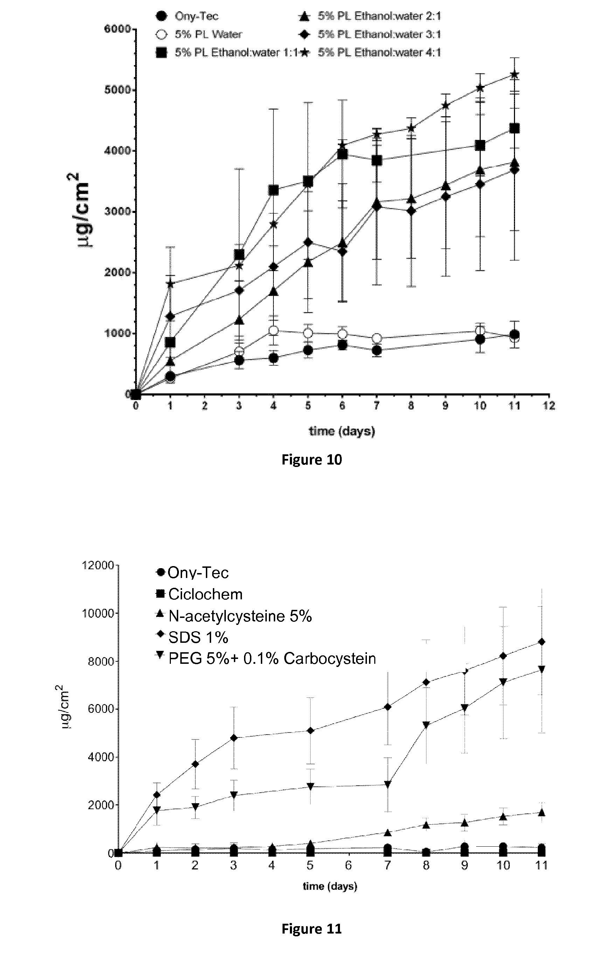

**Figure 10** shows the influence of the etanol:water ratio used in the preparation of pharmaceutical compositions of the invention (2 ml) on the diffusion of ciclopiroxolamine across bovine hoof membrane. Compared to compositions containing only water as solvent, the presence of ethanol increases the drug diffusion rate in all cases.
**Figure 11** shows the diffusion of ciclopiroxolamine through human nail of the following composition comprising (a) 5% w/w of poloxamer 407, 5% w/w of N-acetylcistein, 10% w/w CRYSMEB, water: ethanol (1:1) and 25 mg/ml ciclopirox olamine; (b) 5% w/w of poloxamer 407, 1% w/w of sodium dodecyl sulphate, 10% w/w of CRYSMEB, water:ethanol (1:1) and 12.83 mg/ml of ciclopirox olamine; (c) 5% w/w of poloxamer 407, 0.1% w/w of carbocystein, 5% w/w of PEG300, 10% w/w of CRYSMEB, water:ethanol (1:1) and 18.79 mg/ml of ciclopirox olamine.

### Detailed Description of the Invention

### Ingredients of the Composition

The compositions of the invention are applied over the nail and/or its surrounding area in need of treatment through any appropriate means, such as brush, spray or sponge only to mention a few non limiting examples. Upon application over the afflicted area, the system forms a hydrogel, i.e. a swollen three-dimensional macromolecular structure in an aqueous medium which is insoluble in these medium, which adheres to the nail and from which the drug penetrates and diffuses into and through the nail.

Thus, it can be said that the composition of the invention is thermosensitive because the poloxamer 407 undergoes a transition from solution to gel at temperatures close to the body temperature, forming structured and consistent hydrogels.

Poloxamer 407 according to the present invention is a block copolymer of the general formula (I)

HO(C₂H₄O)ₐ((CH₃)₂CHO)_{b}(C₂H₄O)ₐH (I)

wherein each "a" is independently chosen from an integer comprised between 80 and 115, and "b" from an integer comprised between 45 and 75. It is thus a triblock co-polymer with a central oxypolyisopropylene block, flanked by two oxipolyethylene blocks. In an alternative embodiment, "a" is an integer comprised between 95 and 105, or between 97 and 104. According to a further embodiment, "a" is an integer comprised between 98 and 103. In an alternative embodiment, "b" is an integer comprised between 52 and 65, or between 54 and 60. According to a further embodiment, "a" is an integer comprised between 54 and 58. Typical molecular weights of poloxamer 407 are comprised between 9.500 and 14.600, with an oxipolyethylene content of between 65 to 75% by weight with respect to the total weight of the poloxamer 407.

The exact number of "a" and "b" may vary depending on the specific commercial poloxamer 407 used. For example, BASF commercializes Pluronic^{®} F 127 NF, in which "a" is typically 101 and "b" typically 56, thus having an approximate molecular weight of 12.600 and an approximate oxipolyethylene content of 70% by weight with respect of the total weight of the polymer. The melting point of this commercial poloxamer 407 is 56ºC and the solubility in water at 25ºC above 10%.

According to the present invention, the term "penetration enhancer" refers to a substance used to enhance and facilitate the penetration and diffusion of biologically active substances through the nail matrix. Depending on the mechanism for enhancing nail drug penetration "penetration enhancer" can be classified into:
- Keratolytic enhancers: hydrate and swell the nail plate, such as urea, salicilic acid, thioglycolic acid
- Keratolytic enzymes: hydrolyses the keratin as the keratinases
- Keratin sulfhydryl bridge bond reducing agents such as cysteine, N-acetylcysteine, carbocysteine, sodium sulfite
- Surfactant: some ionic surfactants as sodium dodecyl sulfate, interacts with keratins (mainly via cystin residues) producing changes in the configuration and state of aggregation of this proteins.

One embodiment of the invention comprises penetration enhancers selected from the group of keratin sulfhydryl bridge bond reducing agents, such as cysteine, N-acetylcysteine or carbocysteine. According to a further embodiment, the penetration enhancer is selected from the group consisting of lactic acid, sodium dodecyl sulfate (also known as sodium dodecyl sulfate, and abbreviated as SDS, SLS or DSS) and potassium phosphate. Other useful penetration enhancers are polyethyleneglycols (PEG), especially low molecular weight (i.e. with molecular weight below 1000), such as PEG 300 or 400. Further useful non-limiting penetration enhancers are thioglycolic acid, sodium sulfite, keratinase, hydrogen peroxide, urea and mixtures thereof. In a particular embodiment, N-acetylcysteine has been selected as penetration enhancer.

The authors have also found that specific enhancers provide even better diffusion of active ingredients through human nail when they are incorporated to the formulations of the inventions. According to an embodiment of the invention, the penetration enhancer is sodium lauryl sulfate (also known as sodium dodecyl sulfate) in amounts between 0.01% and 10% w/w, preferably between 1% and 8% w/w, more preferably between 2% and 6% w/w.

Penetration enhancers according to the present invention are not limited to a single compound and can comprise a combination of two or more. An exemplary penetration enhancer comprising two compounds is PEG combined with carbocysteine, for example, the first being present in amounts ranging from 2 to 7% w/w and the second from 0.01% to 0.2% w/w, which provide an excellent diffusion profile in human nail.

In a particular embodiment, polyethyleneglycols have been selected as penetration enhancers.

The composition of the present invention further comprises a solubilizating agent selected from cyclodextrins, hydrophilic polymers and mixtures thereof. Solubilizing agents increase the aqueous solubility of biologically active substances. The addition of solubilizing agents can increase the effective dose of biologically active substances in the system reducing the resistance of aqueous layer diffusion. This issue is however dependent on many factors, one being the molecular weight of the active ingredient (Y. Kobayashi, T. Komatsu, M. Sumi, S. Numajiri, M. Miyamoto, D. Kobayashi, K. Sugibayashi, Y. Morimoto. In vitro permeation of several drugs through the human nail plate: relationship between physicochemical properties and nail permeability of drugs. Eur J Pharm Sci, 21 (2004) 471-477), and cyclodextrins as well as hydrophilic polymers have shown an extraordinary improvement in the diffusion and penetration in combination with aqueous poloxamer 407 (EP 2 567 710).

Cyclodextrins are composed of 5 or more [alpha]-D-glucopyranoside units linked 1->4. The largest well-characterized cyclodextrin contains 32 1,4-anhydroglucopyranoside units, while as a poorly characterized mixture, at least 150-membered cyclic oligosaccharides are also known. Typical cyclodextrins contain a number of glucose monomers of six ([alpha]-), seven ([beta]-) or eight ([gamma]-) units, creating a cone shape.

Cyclodextrins used in the pharmaceutical composition of the invention are preferably selected from the group consisting of [alpha]-, [beta]-, and [gamma]-cyclodextrins and their mixtures. [alpha]-, [beta]-, and [gamma]-alkyl-cyclodextrins and their mixtures; [alpha]-, [beta]-, and [gamma]-hydroxyalkyl-cyclodextrins, such as, for example hydroxyethyl-[beta]-cyclodextrin, 3- or 2-hydroxypropyl-[beta]-cyclodextrin, and hydroxypropyl-[gamma]-cyclodextrin, and their mixtures are also appropriate. [alpha]-, [beta]-, and [gamma]-sulfoalkyl-ether cyclodextrins, such as, for example, sulfobutylether-[beta]-cyclodextrin and their mixtures can also be used in the present invention. [alpha]-, [beta]-, and [gamma]-branched cyclodextrins with one or two glucosyl or maltosyl residues and their mixtures are further embodiments of the invention. Further examples are [alpha]-, [beta]-, and [gamma]-alkylcarboxyalkyl-cyclodextrins and their mixtures.

The term "alkyl" refers to a C₁-C₆ lineal or branched alkyl group, such as methyl, ethyl, propyl, isopropyl or pentyl. The term "hydroxyalkyl" refers to a C₁-C₆ lineal or branched alkyl group substituted by one or two hydroxyl groups, such as 2-hydroxyethyl, 3-hydroxypropyl, or 5-hydroxypentyl. The term sulfoalkyl refers to a -SO₃H group attached to the rest of the molecule through an alkyl group, wherein said alkyl group is a C₁-C₆ lineal or branched alkyl group, such as methyl, ethyl, propyl, isopropyl or pentyl. The term alkylcarboxyalkyl refers to a group of formula alkyl-C(=O)O-alkyl wherein each alkyl is independently a C₁-C₆ lineal or branched alkyl group, such as methyl, ethyl, propyl, isopropyl or pentyl. In a more particular embodiment, the solubilizing agent is a hydroxyalkyl-cyclodextrin, such as hydroxypropyl-beta-cyclodextrin. According to an alternative embodiment the solubilizing agent is a partially methylated cyclodextrin. Cyclodextrins can be purchased in different forms and grades. For example, different cyclodextrins can be obtained with different methylation such as, full methylation or a methylation of 1 to 20, typically 2 to 10, methyl groups per cyclodextrin molecule, i.e. number of groups per mol of cyclodextrin.

In a particular embodiment, hydrophilic polymers are selected from poloxamers, poloxamines, urea, polyethylene glycols, polyvinylpyrrolidone, polysorbates or polyvinyl alcohol. In a further embodiment of the invention the hydrophilic polymers are selected from the group consisting of poloxamines, urea, polyethylene glycols, polyvinylpyrrolidone, polysorbates or polyvinyl alcohol

Poloxamers suitable as hydrophilic polymers are poloxamer 124, 188, 181 or 908. They can be used to increase the spreading and humectation of the formulations or increase the water solubility of hydrophobic drugs. Additionally, varieties of poloxamer such as 188 or 124 can be used in mixtures with poloxamer 407 for modulating and optimizing the sol/gel transition temperature or to increase their bioadhesive properties.

Additionally the solution can also contain other physiologically acceptable additives such as acids, bases, antioxidans (e.g. EDTA), solvents which may accelerate drying (although in a preferred embodiment they are not needed) and pH buffering systems.

The vehicle used in the present invention comprises water and a C₁-C₃ alcohol. Contrary to established consensus against the use of organic solvents due to decreased nail hydration, the inventors have found that the use of a C₁-C₃ alcohol improves diffusion and penetration into the nail. Such improvement is observable for a wide range of proportions between them, for example, when the water: C₁-C₃ alcohol volume proportion is comprised between 20:1 and 1:20. According to a particular embodiment, water: C₁-C₃ alcohol volume proportion is comprised between 10:1 and 1:10, more particularly 5:1 and 1:5, more particularly between 4:1 and 1:4, preferably between 2:1 and 1:2. According to a particular embodiment water: C₁-C₃ alcohol volume proportion is 1:1. According to a preferred embodiment the volume proportion of water is greater or equal than that of C₁-C₃ alcohol, e.g. a water: C₁-C₃ alcohol volume proportion comprised between 20:1 and 1:1, alternatively between 10:1 and 1:1, more preferably between 5:1 and 1:1. According to a particular embodiment the vehicle of the invention consists of water and C₁-C₃ alcohol. The vehicle of the present invention surprisingly results on an improved penetration and diffusion to and into the nail plate, with respect to the water alone.

The enhanced properties of the pharmaceutical composition of the invention are realized over a wide range of proportions among its components. Small variations within the scope of the invention might be required for specific active ingredients. By "% w/w" it is meant the weight percentage of a particular component with respect of the total weight of the pharmaceutical composition, except the biologically active substance. In a particular embodiment of the invention the hydroalcoholic pharmaceutical composition comprises up to 40% w/w of poloxamer 407. In a further embodiment the pharmaceutical composition comprises between 1% and 40% w/w of poloxamer 407. Good results are also obtained by use of between 3 and 25% w/w. Preferred proportions of poloxamer 407 are between 3 and 20% w/w, preferably between 4 and 12 % w/w. Typical amounts of poloxamer 407 are between 5 and 10% w/w.

The solubilizating agent is typically present in amounts up to 30% w/w, generally between 1% and 25% w/w, preferably between 5% and 20% w/w, typically up to 20% w/w. Thus, according to a preferred embodiment the pharmaceutical composition of the invention comprises up to 20% of cyclodextrin (or a mixture of two or more cyclodextrins), more preferably between 5% and 20% w/w.

The penetration enhancer is present in the pharmaceutical composition of the invention in amounts ranging between 0.01% and 15% w/w, preferably between 5% and 12% w/w.

The amount of biologically active substance added to the solution is sufficient to deliver the desired therapeutic effect and is determined by the skilled person. The biologically active substance is typically added to saturation.

A further alternative embodiment of the invention is a pharmaceutical composition comprising (i) up to 40% w/w of poloxamer 407, (ii) up to 15% w/w of at least one penetration enhancer, (iii) up to 30% w/w of at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof in sufficient amount to reach the 100% w/w.

A further alternative embodiment of the invention is a pharmaceutical composition comprising (i) between 5% and 25% w/w of poloxamer 407, (ii) between 0.01% and 12% w/w of at least one penetration enhancer, (iii) between 5% and 25% w/w of at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof in sufficient amount to reach the 100% w/w.

A further alternative embodiment of the invention is a pharmaceutical composition comprising (i) up to 40% w/w of poloxamer 407, (ii) up to 15% w/w of at least one penetration enhancer selected from the group consisting of N-acetylcysteine, carbocysteine, sodium dodecyl sulphate, lactic acid, potassium phosphate, polyethyleneglycols and mixtures thereof (iii) up to 30% w/w of at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof in sufficient amount to reach the 100% w/w.

A preferred embodiment of the invention is a pharmaceutical composition comprising (i) between 3% and 25% w/w of poloxamer 407, (ii) at least one penetration enhancer selected from the group consisting of between 0.01% and 1% w/w of carbocysteine, between 0.5 and 5% w/w of sodium dodecyl sulphate, between 1% and 10% w/w of polyethyleneglycols, and mixtures thereof (iii) between 5% and 25% w/w of cyclodextrins, (iv) at least one biologically active substance, and (v) a vehicle comprising water and a C₁-C₃ alcohol, or mixtures thereof in sufficient amount to reach the 100% w/w.

A particular embodiment of the invention is a pharmaceutical composition comprising (i) 5% w/w of poloxamer 407, (ii) 0.1% w/w of carbocysteine, (iii) 5% w/w of polyethyleneglycol 300, (iv) 10% w/w of methyl-beta-cyclodextrin, (v) at least one biologically active substance, and (vi) a vehicle comprising water and ethanol, in sufficient amount to reach the 100% w/w. A particular embodiment of the invention is a pharmaceutical composition comprising (i) 5% w/w of poloxamer 407, (ii) 0.1% w/w of carbocysteine, (iii) 5% w/w of polyethyleneglycol 300, (iv) 10% w/w of hydroxypropyl-beta-cyclodextrin (HPB), (v) at least one biologically active substance, and (vi) a vehicle comprising water and ethanol, preferably in a 1:1 proportion w/w, in sufficient amount to reach the 100% w/w.

Another particular embodiment of the invention is a pharmaceutical composition comprising (i) 5% w/w of poloxamer 407, (ii) 1% w/w of sodium dodecyl sulphate, (iii) 10% w/w of methyl-beta-cyclodextrin, (iv) at least one biologically active substance, and (v) a vehicle comprising water and ethanol, preferably in a 1:1 proportion w/w,in sufficient amount to reach the 100% w/w.

Another particular embodiment of the invention is a pharmaceutical composition comprising (i) 5% w/w of poloxamer 407, (ii) 1% w/w of sodium dodecyl sulphate, (iii) 10% w/w of hydroxypropyl-beta-cyclodextrin (HPB) (iv) at least one biologically active substance, and (v) a vehicle comprising water and ethanol, in sufficient amount to reach the 100% w/w.

### Preparation of the Composition

A further aspect of the invention is a method for the preparation of the pharmaceutical composition of the invention. It comprises dispersing or dissolving the poloxamer 407, penetration enhancer, the solubilizing agent and at least one biologically active substance in water and in a C₁-C₃ alcohol.

The method is based on the dispersion and dissolution of the components in the aqueous medium. The method involves a single stage and does not have environmental or toxicological problems associated with the use of organic solvents neither the presence of residues of these solvents in the final product. The dispersion or dissolution of the components does not require any particular order and can be prepared according to known procedures such as those described in EP 2 567 710, which, despite the differences in the vehicle used, describes appropriate methods for the synthesis of the pharmaceutical compositions of the invention. Usually, first a dispersion of poloxamer 407 is formed in cold water/alcohol, followed by the addition of the solubilizing agent and then the other components.

### Pharmaceutical Activity

The composition of the invention incorporates a biologically active substance and is used as a medicament, concretely for the treatment of ungual conditions.

The term "biologically active substance" refers to any substance that is used to treat, cure or prevent conditions, e.g. fungal infections of the nails, nail psoriasis and other diseases of the nails such as atopic dermatitis or lichen planus. When one or more biologically active substances are incorporated to the pharmaceutical compositions of the invention, those are dispersed at the molecular level, particle level, forming complexes with components of the solution or included into systems to improve their solubility or control their release. The system of the invention is suitable for incorporating biologically active substances regardless their solubility properties.

The pharmaceutical composition of the invention is applied to the nail, which may include the nail plate (the *stratum corneum unguis*) but may also exert its pharmaceutical action on the nail bed (the modified area of epidermis beneath the nail, over which the nail plate slides as it grows). It can also be concurrently administered to the nail matrix (i.e. the proximal portion of the nail bed from which growth proceeds) and over the cuticle and the *hyponychium* (the thickened epidermis underneath the free distal end of the nail).

Non-limiting examples of antifungal agents that can be employed in the pharmaceutical composition of the invention are cyclopirox, amphotericin B, flucytosine, fluconazole, griseofulvin, miconazole nitrate, terbinafine hydrochloride, ketoconazole, itraconazole, undecylenic acid and chloroxylenol, ciclopirox, clotrimazole, butenafine hydrochloride, nystatin, naftifine hydrochloride, oxiconazole nitrate, selenium sulfide, econazole nitrate, terconazole, butoconazole nitrate, carbol-fuchsin, clioquinol, methylrosaniline chloride, sodium thiosulfate, sulconazole nitrate, tioconazole, tolnaftate, voriconzole, undecylenic acid, and undecylenate salts.

Non-limiting examples of anti-inflammatory drugs that can be employed in the pharmaceutical compositions of the invention are aspirin, ibuprofen, naproxen, diclofenac, ketoprofen, flubiprofen, and Cox-2 anti-inflammatory agents such as rofecoxib, celecoxib, etoricoxib, valdecoxib and lumiracoxib.

Non-limiting examples of steroidal anti-inflammatory drugs that can be employed in the pharmaceutical compositions of the invention are clobetasol, triamcinolone, prednisone, prednisolone, methylprednisolone, betamethasone, dexamethasone, hydrocortisone, Fluocinonide, Flurandrenolide, Halobetasol, Amcinonide, Desoximetasone, Diflorasone, Halocinonide, Diflorasone, Mometasone, Clocortolone, Desonide, Fluticasone, Hydrocortisone, Prednicarbate, Aclometasone.

Non-limiting examples of retinoid drugs that can be employed in the pharmaceutical compositions of the invention are retinol, retinaldehyde, Tretinoin, Isotretinoin, Alitretinoin, Alfa-14-hydroxy-retro-retinol, fenretinide, polyprenoic acid, etretinate, acitretin, Isoacetritenol, motretinide, poloprenoic acid arotinoid ethyl ester, Arotinoid carboxylic acid, Arotinoid ethyl sulfona, Arotinoid methyl sulfona, Adapalene, Tazarotene, Bexarotene.

Non-limiting examples of vitamin D analogues that can be employed in the pharmaceutical compositions of the invention are calcitriol , Calcipotriol, Maxacalcitol and Tacalcitol. Non-limiting examples of drug for photodynamic therapy that can be employed in the pharmaceutical compositions of the invention are 8-metoxipsoralen, 5-aminolevulinic acid and methylaminolevulinate.

Non-limiting examples of antibiotics that can be employed in the pharmaceutical composition of the invention are cefadroxil, cefazolin, cephalexin, cephalothin, cephapirin, cephacelor, cephprozil, cephadrine, cefamandole, cefonicid, ceforanide, cefuroxime, cefixime, cefoperazone, cefotaxime, cefpodoxime, ceftaxidime, ceftibuten, ceflizoxime, ceftriaxone, cefepime, cefinetazole, cefotetan, cefoxitin, loracarbef, imipenem, erythromycin (and erythromycin salts such as estolate, ethylsuccinate, gluceptate, lactobionate, stearate), azithromycin, clarithromycoin, dirithromycin, troleanomycin, penicillin V, penicillin salts, and complexes, methicillin, nafcillin, oxacillin, cloxacillin, dicloxacillin, amoxicillin, amoxicillin and clavulanate potassium, ampicillin, bacampicillin, carbenicillin indanyl sodium (and other salts of carbenicillin) mezlocillin, piperacillin, piperacillin and taxobactam, ticarcillin, ticarcillin and clavulanate potassium, clindamycin, vancomycin, novobiocin, aminosalicylic acid, capreomycin, cycloserine, ethambutol HCl and other salts, ethionamide, and isoniazid, ciprofloxacin, levofloxacin, lomefloxacin, nalidixic acid, norfloxacin, ofloxacin, sparfloxacin, sulfacytine, suflamerazine, sulfamethazine, sulfamethixole, sulfasalazine, sulfisoxazole, sulfapyrizine, sulfadiazine, sulfmethoxazole, sulfapyridine, metronidazole, methenamine, fosfomycin, nitrofurantoin, trimethoprim, clofazimine, co-triamoxazole, pentamidine, and trimetrexate.

Non-limiting examples of antiviral agents that can be employed in the composition of the invention are acyclovir, amantadine, amprenavir, cidofovir, delavirdine, didanosine, famciclovir, foscamet, ganciclovir, indinavir, interferon, lamivudine, nelfinavir, nevirapine, palivizumab, penciclovir, ribavirin, rimantadine, ritonavir, saquinavir, stavudine, trifluridine, valacyclovir, vidarabine, zalcitabine, and zidovudine.

A preferred agent for the treatment of psoriasis is clobetasole propionate.

In a preferred embodiment the biologically active substance is an antifungal agent. In a more preferred embodiment the active ingredient is an antifungal agent that is suitable for treating onychomycosis. In a particularly preferred embodiment the active ingredient is cyclopirox or terbinafine.

The amount of biologically active substance in the pharmaceutical composition of the invention is sufficient to deliver a "therapeutically effective amount", that is, a dose regimen, or treatment protocol, or combination thereof, that achieves a successful treatment effect. Such amounts depend on the particular biologically active substance used, but can be, for example, a load of from 0.01 to 100 mg of biologically active substance per ml of pharmaceutical composition of the invention (mg/ml), preferably, between 5 and 30 mg/ml.

The pharmaceutical compositions of the invention enhance the penetration and diffusion into the nail of a therapeutically effective amount of a biologically active substance. The term "nail" may include the nail bed, nail matrix and/or nail plate. It may be thus intended and designed to enhance delivery of a therapeutically effective amount of a biologically active substance to a diseased or infected nail bed, nail matrix, and/or nail plate in the toenails and/or fingernails of a patient. In a preferred embodiment said disease or infection is onychomycosis. In an alternative embodiment, the condition is psoriasis.

It is usually advantageous to provide the maximum possible concentration of the biologically active substance in the composition. This is easily determined by preparing oversaturated compositions of the biologically active substance (with stirring for long periods) and then measurement of the biologically active substance concentration (e.g. se example 2 of EP 2 567 710).

In a particular embodiment, the biologically active substances are selected from steroidal anti-inflammatory and antifungal drugs.

In a particular embodiment, the antifungal drug is selected from the group consisting of polyenes, allylamines, imidazoles, triazoles such as econazole, ciclopirox, undecylenic acid and amorolfine, and their salts.

In another particular embodiment, the steroidal anti-inflammatory drug is selected from the group consisting of hydrocortisone, triamcinolone, betamethasone, clobestol, and their salts.

In a further embodiment of the invention said biologically active substance is selected from the group consisting of clobetasole propionate, cyclopirox and terbinafine.

The pharmaceutical composition can be applied to the nails by deposition, spraying, atomization, misting and / or immersion.

### Examples

### Materials and Methods

Ciclopirox olamine was provided by Fagron Iberica and Clobetasol Propionate by Crystal Farma. Partially methylated cyclodextrine (Kleptose^{®} CRYSMEB EXP) and HPB (Kleptose^{®} HPB) is a gift from Roquette. It is a mixture of several [beta]-cyclodextrins (d-glucopiranose (glucose), bonded by [alfa]-1-4 bonds) with 1 to 7 methyl groups (in secondary hydroxyl groups), in average four, and having molar substitution ratio (MS) of 0.57. Average molecular weight is 1191 (Mw= 1135 + 7xMSx14). N-acetylcisteine is a gift from Acorfarma. Sodium dodecyl sulphate was provided by Fagron Iberica, which is also known as sodium lauryl sulphate. The saline phosphate buffer was prepared according to Spanish Farmacopeia from potassium dihydrogen phosphate, sodium chloride and from sodium dihydrogen phosphate dodecahydrate, all of analytic grade. Sodium azide added to the buffer to prevent microbial growth was supplied by Panreac Quimica SA (Barcelona, Spain). The rest of solvents and reagents used have analytic grade.

### Example 1: General Methods

### Example 1.1: General Method for Preparing Formulations According to the invention

With the exception of Ony-Tec, the formulations described below were prepared of according to the following method.

The required amount of cyclodextrin .(CRYSMEB or HPB) was dissolved in the corresponding vehicle, under constant stirring and a temperature of about 4°C to promote dissolution and correct homogenization of poloxamer, which was incorporated once cyclodextrin was dissolved. Once cyclodextrin and the poloxamer were completely dissolved, and maintaining the conditions of low temperature and constant stirring, the penetration enhancer was added (e.g. N -acetylcysteine, carbocysteine or sodium dodecyl sulfate), and later the biologically active substance to saturation when the formulation was homogeneous and clear. Stirring was continued overnight at room temperature. In the case of high concentrations of poloxamer 407 it was necessary to maintain the temperature at 4-6°C. Saturated solutions of biologically active substance were filtered the next day (filters of 0.45 microns of material compatible with the solvent used in each case). The concentration of the biologically active substance was determined by UV spectrophotometry or HPLC.

### Example 1.2: Pre-treatment of nails/hoofs

Samples were obtained of nails from the hands and feet of healthy volunteers and patients (with onychomycosis and psoriasis) aged between 25 and 65. Healthy volunteers cut their own nails after informed consent. The samples were carefully cleaned and washed with water, dried at room temperature and stored in a glass vessel at room temperature. The nails used for diffusion studies had a length of about 8 mm.

Bovine hoofs used were obtained from the municipal slaughterhouse of Santiago of Compostela. They were cleaned and washed with water and were kept frozen. Prior to use they were thawed and kept in water for 24 hours to facilitate cutting thin slices of approximately 0.3-0.8 mm of thickness.

### Example 1.3: Diffusion Studies of the Biologically Active Substance

Diffusion Studies described in this application were conducted using the method detailed below.

Diffusion Studies through human nail and bovine hoof were conducted by placing the samples between two cylindrical adapters made of Teflon, with an effective surface area of diffusion of 0.196 cm². These adapters were placed between the donor and the acceptor of Franz diffusion cells. The dorsal part of the nail was placed to bring it into contact with the formulation studied in the donor compartment, and the ventral part with the receptor compartment's media, which consisted of a saline solution of phosphate buffer pH 7.4, (European Pharmacopoeia) at 37°C, to which was added 0.003% of sodium azide to prevent the growth of algae and microorganisms. At preset periods, samples were taken from the acceptor compartment, keeping the volume constant with replenishment of fresh medium.

In the case of clobetasol propionate the acceptor compartment included 5% w/w of CRYSMEB with 0.003% of sodium azide. The incorporation of CRYSMEB aims to maintain sink conditions and prevent the acceptor from saturating, thus limiting and even preventing the passage of the biologically active substance through the nail.

The concentration of the biologically active substance was determined for each substance with suitable analytical techniques (UV spectrophotometry, HPLC). In the case of the formulations with ciclopirox olamine tested in human nail and analyzed by UV spectroscopy, samples were diluted with NaOH to minimize interference of nail components.

### Example 1.4: "in vitro" Penetration Studies

The amount of biologically active substance that had penetrated the nail after completion of the diffusion tests was also determined. The nail was recovered after the diffusion test, which was thoroughly washed with water and wiped with cellulose paper. The section that had been exposed to the formulation was cut into small pieces and weighed. 5 ml of a 5% methanol solution was added and incubated for at least 6 days at room temperature.

### Example 1.5: Release Studies

They were carried out in Franz vertical diffusion cells having an effective diffusion area of 0.79 cm². In the donor compartment was added, unless otherwise stated, 500 µl of the formulation to be studied and the acceptor compartment was formed by a saline solution of phosphate buffer pH 7.4 (European Pharmacopoeia) at 37°C and under continuous stirring, separated from the acceptor compartment by a dialysis membrane of MWCO > 12,000 Da. Samples were collected at stipulated intervals of time from the acceptor compartment, replenishing the volume with phosphate buffer. The biologically active substance concentration was determined spectrophotometrically by diluting the samples as needed.

### Example 2: Release Study of Ciclopirox Olamine

Following the methodology described in Example 1.5, the release of ciclopirox olamine in formulations prepared with different ratios of poloxamer 407 containing 10% of CRYSMEB and 10% of N-acetylcysteine was studied by comparing aqueous compositions (comparative) with a vehicle mixture of water and ethanol 1:1 (compositions of the invention). As shown in Figure 1, the incorporation of ethanol increases diffusion ciclopirox olamine.

### Example 3: Penetration Study and Diffusion of Ciclopirox Olamine in Bovine Hoof

Following the methodology described in Example 1.3 diffusion and penetration of ciclopirox olamine through of bovine hoof was studied in compositions having poloxamer 407 in 5% w/w, 10% w/w N-acetylcistein and 10% w/w CRYSMEB. Figure 2 shows diffusion profiles obtained with these formulations. It is observed that the comparative formulations made with water and the Ony-Tec possess similar diffusion profiles. However, the composition of the invention using hydroalcoholic solution (water:ethanol 1:1) leads to surprisingly superior drug diffusion through the hoof. The determination of the amount of ciclopirox olamine present inside the hoof at the end of the 11-day trial were also found to be greater with the formulations of the invention (Figure 3).

### Example 4: Penetration Study in Human Nail

Two formulations were prepared according to the invention: (a) 5% w/w of poloxamer 407, 10% w/w of N - acetylcistein, 10% w/w CRYSMEB, water: ethanol (1:1) and 24.27mg/ml ciclopirox olamine, and (b) 10% w/w of poloxamer 407, 10% w/w of N- acetylcistein, 10% w/w of CRYSMEB, water:ethanol (1:1) and 22.90 mg/ml of ciclopirox olamine.

The study was performed on human nail following the methodology described in Example 1.3, but in this case, due to rapid diffusion, after applying the initial dose of 0.5 ml, the same amount was further added after 5 days of testing. As shown in Figure 4, the two compositions (a) and (b) have greater penetration in the nail than Ony-Tec, the formulation (a) with 5% w/w of poloxamer 407 being particularly advantageous.

The same experiment was repeated by comparing solution (a) with Ony-Tec, but putting 2ml of solution on the plate to ensure sufficient amount of ciclopirox olamine in formulations throughout the test. This is equivalent to doses of 47 mg of ciclopirox olamine in formulation (a) according to the invention and of 160 mg for Ony-Tec. The results shown in Figures 5 and 6 confirm a significant improvement in both, diffusion (Figure 5), and penetration (Figure 6) of ciclopirox olamine in the nail when formulations of the present invention were used.

### Example 5: Diffusion and Penetration Study in Formulations of the Invention containing Clobetasol

A formulation (d) was prepared according to the present invention comprising 5% w/w of poloxamer 407, 10% w/w of N-acetylcistein, 10% w/w of CRYSMEB, water: ethanol (1:1), and 1.6 mg/ml of clobetasol propionate. In the absence of commercial formulations, a formulation was prepared with a base lacquer of Fagron incorporating 80 mg of clobetasol propionate.

A study of diffusion following the general procedure described in Example 1.3 was performed on bovine hoof. The study shows that the formulation of the invention (d) provides significantly higher values of diffusion (Figure 7). The same experiment was repeated under the same conditions on human nail, providing a similar results (Figure 8). The constant flow of clobetasol propionate was a total of 20 mg/cm² during the 11 days, far more than necessary for effective treatment.

### Example 6: Diffusion Study through Bovine Hoof of Formulations According to the Invention Comprising Different Penetration Enhancers

Except for the Ony-Tec composition, various formulations were prepared following the general procedure described in Example 1.1 using 5% w/w of poloxamer 407, 10 % w/w of CRYSMEB, ciclopiroxolamine to saturation, water:ethanol 1:1 to complete 100% and different penetration enhancers in different proportions. Following the general procedure described in example 1.3 formulations were compared in all cases with Ony-Tec. Results are shown in Figures 9a-e. Figure 9a shows results of carbocysteine. Figure 9b shows the results of Sodium dodecyl sulfate (SDS). Figure 9c shows the results of potassium phosphate. Figure 9d shows the results of lactic acid. Figure 9e shows the results of polyethyleneglycol 300 (PEG300) with 0.1 % w/w of carbocystein. Pharmaceutical compositions according to the invention having N-acetyl-cisteine (Ac) were used as reference. Good diffusion was observed for all penetration enhacers, even carbocystein which despite lack of smell is many times discarded because of its poor solubility.

### Example 7: Diffusion Study with Different Proportions of water and Alcohol

Except for the Ony-Tec composition, various formulations were prepared following the general procedure described in Example 1.1 using 5% w/w of poloxamer 407, 5% w/w of CRYSMEB, penetration enhancer, ciclopiroxolamine to saturation and different proportions of water:ethanol to complete 100%. Following the general procedure described in example 1.3 formulations were compared in all cases with Ony-Tec and the same composition of the invention using only water. Influence of the etanol:water ratio used in the elaboration of the nail lacquer (2 ml) on the diffusion of ciclopiroxolamine across bovine hoof membrane. In comparison to lacquers containing only water as solvent, presence of ethanol increases the drug diffusion rate in all cases (See Figure 10).

### Example 8: Diffusion Study in Human Nail

Three formulations were prepared according to the invention: (a) 5% w/w of poloxamer 407, 5% w/w of N-acetylcistein, 10% w/w CRYSMEB, water: ethanol (1:1) and 25 mg/ml ciclopirox olamine; (b) 5% w/w of poloxamer 407, 1% w/w of sodium dodecyl sulfate SDS), 10% w/w of CRYSMEB, water:ethanol (1:1) and 12.83 mg/ml of ciclopirox olamine; (c) 5% w/w of poloxamer 407, 0.1% w/w of carbocystein, 5% w/w of PEG300, 10% w/w of CRYSMEB, water:ethanol (1:1) and 18.79 mg/ml of ciclopirox olamine.

The study was performed on human nail following the methodology described in Example 1.3. As shown in Figure 11, the three compositions (a), (b) and (c) have greater penetration in the nail than Ony-Tec and Ciclochem. The formulations (b) and (c) with sodium dodecyl sulfate and PEG300 are particularly advantageous.

## Claims

1. A pharmaceutical composition comprising (i) poloxamer 407, (ii) at least one penetration enhancer, (iii) at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof.

2. The pharmaceutical composition according to claim 1, wherein the water: C₁-C₃ alcohol proportion is comprised between 20:1 and 1:20.

3. The pharmaceutical composition according to claim 2, wherein the water: C₁-C₃ alcohol proportion is comprised between 4:1 and 1:4.

4. The pharmaceutical composition according to any of the previous claims, wherein said vehicle consists of a mixture of water and C₁-C₃-alcohol.

5. The pharmaceutical composition according to any of the previous claims, comprising a cyclodextrin in up to 20% w/w with respect to the total weight of the pharmaceutical composition.

6. The pharmaceutical composition according to any of the previous claims, comprising between 1% and 40% w/w with respect to the total weight of the pharmaceutical composition of poloxamer 407.

7. The pharmaceutical composition according to any of the previous claims, wherein said penetration enhancer is selected from the group consisting of N-acetylcisteine in an amount between 0.01% and 15% w/w, sodium dodecyl sulfate in an amount between 0.01% and 10% w/w, PEG in an amount between 2% to 7% w/w and a combination of PEG in an amount between 2% to 7% w/w with carbocysteine in an amount between 0.01% to 0.2% w/w.

8. The pharmaceutical composition according to claim 1, comprising (i) between 5% and 25% w/w of poloxamer 407, (ii) between 0.01% and 12% w/w of at least one penetration enhancer, (iii) between 5% and 25% w/w of at least one solubilizing agent selected from the group consisting of cyclodextrins, derivates thereof, hydrophilic polymers and mixtures thereof, (iv) at least one biologically active substance to saturation, and (v) a vehicle comprising water and a C₁-C₃ alcohol, or mixtures thereof in an amount to complete 100% w/w.

9. The pharmaceutical composition according to any of the previous claims, wherein said biologically active substance is selected from the group consisting of antifungals, steroidal antiinflammatories, corticoids, retinoids, vitamin D and derivatives thereof, immunosuppressants, antivirals, antibiotics and mixtures thereof.

10. The pharmaceutical composition according to claim 9, wherein said biologically active substance is selected from the group consisting of clobetasole propionate, cyclopirox and terbinafine.

11. A pharmaceutical composition as defined in any of claims 1 to 10 for use as a medicament.

12. A pharmaceutical composition as defined in any of claims 1 to 10 for use in the treatment and prevention of ungual conditions.

13. The pharmaceutical composition according to claim 12, for use in the treatment of a condition selected from the group consisting of fungal infections, psoriasis, lichen planus, inflammation, atopic dermatitis, eczema, and viral and bacterial infections.

14. A Method for the preparation of a pharmaceutical composition which comprises mixing (i) poloxamer 407, (ii) at least one penetration enhancer, (iii) at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof.

15. A kit comprising a pharmaceutical composition which comprises (i) poloxamer 407, (ii) at least one penetration enhancer, (iii) at least one solubilizing agent selected from the group consisting of cyclodextrins, hydrophilic polymers and mixtures thereof, (iv) at least one biologically active substance, and (v) a vehicle comprising water and a C₁-C₃ alcohol, or mixtures thereof; and instructions for ungual application of said pharmaceutical composition.
